# FASCICULE DE BREVET EUROPEEN

(11) **EP 2 130 530 B1**
(45) Date de publication et mention de la délivrance du brevet: **27.03.2019**
(21) Numéro de dépôt: 09161121.0
(22) Date de dépôt: 26.05.2009
(51) Int. Cl.: A61K 8/44, A61K 8/04, A61Q 9/02, A61Q 19/10

(54) **Gel auto-moussant sans savon a base de n-acylsarcosine ; procede de rasage ; procede de nettoyage**
Schaumbildendes Gel ohne Seife auf Basis von N-Acylsarcosin, Rasierverfahren und Reinigungsverfahren
Soap-free, self-foaming gel based on N-acylsarcosine, shaving method, washing method

(30) Priorité: 02.06.2008 FR 0853599
(43) Date de publication de la demande: 09.12.2009
(73) Titulaire: L'Oréal, 75008 Paris (FR)
(72) Inventeur: Aubert, Lionel, 95270 ASNIERES SUR OISE (FR); Dussault, Lydia, 78860 ST NOM LE BRETECHE (FR)
(74) Mandataire: Miszputen, Laurent

(56) Documents cités:
- EP-A- 1 872 830
- WO-A-02/087520
- US-A- 3 959 160
- US-A1- 2005 112 084

## Description

La présente invention concerne un dispositif de délivrance d'un gel auto-moussant sans savon à base de N-acylsarcosine. Une telle composition est distribuée sous la forme d'un gel contenant un composant volatil qui se transforme en mousse lors de l'étalement sur la peau. Ce produit peut être utilisé comme produit de rasage ou comme produit de nettoyage de la peau

Les gels de rasage auto-moussants sont biens connus et ont été décrits, par exemple, dans les brevets US2,995,521 ; US3541,581, US4,405,489 ; US4,528,111 ; US4,651,503 ; US 5,248,495 ; US5,308,643 ; US5,326,556 et la demande de brevet WO91/07943. De telles formulations se présentent sous la forme d'une émulsion huile-dans-eau dans laquelle l'agent auto-moussant, généralement un hydrocarbure aliphatique volatil (ie : de faible point d'ébullition) est solubilisé dans la phase huileuse et la phase aqueuse comprend un savon hydrosoluble. Le produit est généralement conditionné dans un récipient aérosol avec une séparation telle qu'un piston ou une poche souple pour séparer l'agent auto-moussant de l'agent propulseur nécessaire pour l'expulsion du produit. Le produit est appliqué sous forme de gel transparent, translucide ou opaque qui est subtantiellement sans mousse jusqu'à l'étalement sur la peau, moment à partir duquel il se produit une mousse par évaporation de l'agent moussant hydrocarbure volatil.

Les gels auto-moussants conventionnels sont appréciés par une large gamme de consommateurs. Cependant, ces produits en raison de la présence de savon dans la composition ont tendance à assécher la peau et à augmenter la rugosité de celle-ci. Pour atténuer cet effet, les gels auto-moussants sont formulés avec des agents adoucissants comme des humectants, des émollients, des silicones, etc. L'incorporation de ces additifs influe sur les qualités esthétiques du produit et peut provoquer aussi par usage répété un dessèchement de la peau. Pour ces raisons se sont développés des gels « sans savon » contenant des N-acylsarcosinates.

Les N-acylsarcosinates sont des tensioactifs anioniques bien connus de formule : 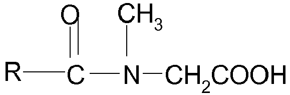 dans laquelle R est une chaîne hydrocarbonée d'acide gras. Ces tensioactifs sont généralement utilisés sous forme de sels hydrosolubles formés par neutralisation avec la soude, la potasse, l'ammoniaque ou la triéthanolamine et ont été préconisés dans une large gamme de produits comme les shampooings, les détergents, les dentifrices, les crèmes à raser ou les savons pour les mains. Par exemple, des crèmes à raser aérosol contenant des sarcosinates sont décrites dans les brevets US3,959,160 ; US4,113,643 et US4,140,648 ainsi que dans le document Harry's Cosmetology (7 ème édition, 1982 page 169 -voir Croda Cosmetic and Pharmaceutical Formulary Supplement, formula SV11). Une crème non aérosol de rasage pouvant contenir un N-acylsarcosinate a été décrite dans le brevet US4,892,729 et un gel de rasage non aérosol contenant un savon et un sarcosinate a été décrit dans le brevet US5,340,571. L'utilisation de ces tensiactifs est également connue dans des bases de nettoyage de la peau et notamment le brevet US6228822.

Pour résoudre les inconvénients énoncés ci-dessus, on a proposé dans le brevet EP782436 des gels de rasage auto-moussants sans savon comprenant 65 à 85% d'eau, 4 à 16% en poids de N-acylsarcosine dans lequel le radical acyle est C₁₀-C₂₀, une base organique ou minérale dans une quantité suffisante pour solubiliser la N-acylsarcosine et produire un pH de 4 à 8, 1 à 8% en poids d'un agent automoussant et 1 à10% en poids d'une hydrocarbure liquide paraffinique non volatil.

On a proposé dans la demande de brevet WO05/094764 des gels de rasage auto-moussants sans savon comprenant 65 à 85% d'eau, 4 à 16% en poids de N-acylsarcosine dans lequel le radical acyle est C₁₀-C₂₀, une base organique ou minérale dans une quantité suffisante pour solubiliser la N-acylsarcosine et produire un pH de 4 à 8, 1 à 8% en poids d'un agent auto-moussant et 0,25 à 5% en poids d'un polyol à chaîne courte en C₃-C₆ (glycérine, propylèneglycol).

Ces formules d'une part nécessitent la présence de N-acylsarcosine à des teneurs supérieures ou égales à 4% pour permettre la formation d'un gel de rigidité suffisante pour une bonne utilisation. D'autre part les qualités de la mousse obtenues lors de l'application sur la peau restent encore insuffisantes.

On a proposé également dans la demande de brevet EP1872830 des gels de rasage auto-moussants sans savon comprenant dans un milieu cosmétiquement acceptable
a) au moins une phase aqueuse
b) au moins une N-acylsarcosine où le radical acyle est en C₁₀-C₂₀ dans une quantité inférieure à 4% en poids par rapport au poids total de la composition ;
c) au moins une base minérale ou organique dans une quantité suffisante pour solubiliser la N-acylsarcosine et produire un pH de 4 à 8 ;
d) au moins un tensioactif anionique ;
e) au moins un tensioactif non-ionique
f) au moins un agent automoussant.

Cependant la rigidité du gel obtenue avec ces formulations n'est pas encore pleinement satisfaisante.

La demanderesse a découvert de manière surprenante que l'on pouvait obtenir des gels auto-moussants sans savon de rigidité améliorée et ayant de bonnes propriétés moussantes sans les inconvénients énoncés précédemment en utilisant une N-acylsarcosine à des teneurs inférieures à 4 % en présence d'une base minérale ou organique dans une quantité suffisante pour solubiliser la N-acylsarcosine et produire un pH de 4 à 8; au moins un tensioactif amphotère ou zwittérionique, au moins un tensioactif non-ionique, au moins un agent auto-moussant.

La demanderesse a également découvert que ces mêmes gels auto-moussants sans savon pouvaient aussi constituer des produits pour le nettoyage de la peau et plus particulièrement le visage ayant de bonnes propriétés détergentes qui ne dessèchent pas la peau et s'éliminent bien au rinçage.

La présente invention concerne donc un dispositif de délivrance d'un gel auto-moussant sans savon comprenant :
(i) un récipient aérosol avec une séparation telle qu'un piston ou une poche souple pour séparer l'agent auto-moussant de l'agent propulseur nécessaire pour l'expulsion du produit
(ii) un tube souple ;
(iii) un flacon pompe ou
(iv) un flacon à paroi déformable, et
un gel auto-moussant sans savon comprenant dans un milieu cosmétiquement acceptable :
a) au moins une phase aqueuse
b) au moins une N-acylsarcosine où le radical acyle est en C10-C20 dans une quantité inférieure à 4% en poids par rapport au poids total de la composition ;
c) au moins une base minérale ou organique dans une quantité suffisante pour solubiliser la N-acylsarcosine et produire un pH de 4 à 8 ;
d) au moins un tensioactif amphotère ou zwittérionique ;
e) au moins un tensioactif non-ionique
f) au moins un agent automoussant choisi parmi les hydrocarbures volatils et les hydrocarbures volatils halogénés de point d'ébullition allant de -20 à 40 °C.

La présente demande décrit un gel auto-moussant sans savon comprenant dans un milieu cosmétiquement acceptable
a) au moins une phase aqueuse
b) au moins une N-acylsarcosine où le radical acyle est en C₁₀-C₂₀ dans une quantité inférieure à 4% en poids par rapport au poids total de la composition ;
c) au moins une base minérale ou organique dans une quantité suffisante pour solubiliser la N-acylsarcosine et produire un pH de 4 à 8 ;
d) au moins un tensioactif amphotère ou zwitterionique ;
e) au moins un tensioactif non-ionique
f) au moins un agent auto-moussant.

La présente invention concerne également un procédé de rasage consistant à appliquer sur la surface de la peau à raser un gel auto-moussant tel que défini précédemment délivré au moyen d'un dispositif de délivrance selon l'invention puis à raser les poils au moyen d'un rasoir.

La présente invention concerne également un procédé de nettoyage de la peau et plus particulièrement du visage consistant à appliquer sur la surface un gel auto-moussant tel que défini précédemment délivré au moyen d'un dispositif de délivrance selon l'invention suivi d'un rinçage à l'eau.

On entend par « sans savon » comme contenant moins de 1% en poids de savon.

On entend par « milieu cosmétiquement acceptable » on entend par compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Les N-acylsarcosines conformes à l'invention sont choisies de préférence parmi celles ayant un radical acyle en C₁₂-C₁₈. Plus préférentiellement, elles sont choisies parmi stéaroyl sarcosine, myristoyl sarcosine, oleoyl sarcosine, lauroyl sarcosine, cocoyl sarcosine ou leurs mélanges. Encore plus préférentiellement, elles sont choisies parmi stéaroyl sarcosine, myristoyl sarcosine et leurs mélanges. La ou les sarcosines sont présents à des teneurs inférieures à 4% en poids et de préférence allant de 2,5 à 3,5% par rapport au poids total de la composition.

Selon une forme particulière de l'invention, on peut utiliser un sarcosinate pré-neutralisé. Dans ce cas, il ne sera pas nécessaire d'ajouter séparément la base à la composition sauf s'il faut ajuster le pH de la composition comme souhaité.

La base peut être choisie parmi les bases minérales comme la potasse, la soude, l'ammoniaque. Elle peut être choisie parmi les bases organiques en particulier les alcanolamines telles que isopropanolamine, mono-, di- et triéthanolamine, aminoethylpropanol et aminomethylpropanol. La triéthanolamine est préférentielle. La quantité de base utilisée dépend de la quantité de sarcosine présente dans la composition. Une quantité suffisante de base doit être utilisée pour solubiliser la sarcosine dans la phase aqueuse et produire un pH de 4 à 8 et plus préférentiellement de 5 à 7. Pour atteindre cette plage de pH, la sarcosine est de préférence neutralisée de 50 à 90%, plus préférentiellement de 60 à 80%. On utilisera de préférence la sarcosine en léger excès molaire par rapport à la base. La base est de préférence présente à un taux variant de 1 à 6% par rapport au poids total de la composition.

La phase aqueuse des compositions convenant à l'invention représente de préférence de 65 à 85% en poids du poids total de la composition et plus préférentiellement de 70 à 80% en poids.

Les tensioactifs amphotères et zwitterioniques conformes à l'invention peuvent être choisi parmi les alkylbétaïnes, les N-alkylamidobétaïnes et leurs dérivés, les sultaïnes, les alkyl-polyaminocarboxylate (APAC), les alkylamphoacétates et leurs mélanges.

Comme alkylbétaïnes, on peut citer par exemple la cocobétaïne comme le produit commercialisé sous la dénomination DEHYTON AB-30® par la société Cognis ou les produits commerciaux MIRATAINE BB/FLA de RHODIA ou EMPIGEN BB/FL de Huntsman ; la laurylbétaïne comme le produit commercialisé sous la dénomination GENAGEN KB® par la société Clariant ou le produit commercialisé sous la dénomination EMPIGEN BB / LS® par la société Hunstma,; la laurylbétaïne oxyethylénée (10 OE), comme le produit commercialisé sous la dénomination LAURYLETHER (10 OE) BETAINE® par la société Shin Nihon Rica ; la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou sous la dénomination EMPIGEN BB® par la société Albright & Wilson ; la lauramidopropyl bétaïne commercialisée sous la dénomination REWOTERIC AMB12P® par la société Witco.

Comme sultaines, on peut citer le cocoyl-amidopropylhydroxy-sulfobetaine commercialisé sous la dénomination CROSULTAINE C-50® par la société Croda.

Comme alkyl-polyaminocarboxylates (APAC), on peut citer le cocoylpolyamino-carboxylate de sodium, commercialisé sous la dénomination AMPHOLAK 7 CX/C®, et AMPHOLAK 7 CX® par la société Akzo Nobel ; le stéaryl-polyamidocarboxylate de sodium commercialisé sous la dénomination AMPHOLAK 7 TX/C® par la société Akzo Nobel ; la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie ; et le N-cocoyl-N-hydroxyéthyl-N-carboxyméthyl-éthylènediamine N-sodique (nom CTFA : sodium cocamphoacetate).

On utilisera plus particulièrement parmi les tensio-actifs amphotères ou zwittérioniques, les alkylbétaines et encore plus préférentiellement la laurylbétaine et plus particulièrement la lauryl bétaine sous forme de solution aqueuse à 30% en mélange avec du chlorure de sodium (nom INCI : Lauryl Betaine (and) Sodium Chloride) tel que le produit commercial EMPIGEN BB/LS de Huntsman.

Les compositions convenant à l'invention contiennent un ou plusieurs tensioactifs non ioniques. Ce sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools gras dont la chaîne grasse comporte de préférence de 8 à 2 atomes de carbone ; les alcools, les alpha-diols, les alkylphénols, les acides gras, les acides gras polyéthoxylés, polypropoxylés ou polyglycérolés, ayant une chaîne grasse comportant, de préférence, de 8 à 20 atomes de carbone, et où le nombre de groupements oxyde d'éthylène ou oxyde de propylène varie de préférence de 2 à 60 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant de préférence en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de préférence de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine ; et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, sous la dénomination PLANTAREN 2000 UP® par la société Cognis, et sous la dénomination ORAMIX NS 10® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit fabriqué sous la dénomination CHIMEXANE NF® par la société Chimex.

Les tensioactifs non-ioniques préférentiels sont choisis parmi :
- les alcools gras ont la chaîne grasse est en C₈-C₂₀, plus préférentiellement dont la chaîne grasse est en C₁₂-C₁₈ comme par exemple l'alcool myristique, l'alcool laurique, l'alcool stéarylique et l'octyldodécanol ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène. Parmi ces composés, on peut citer par exemple Oleth-30, Steareth-21, Ceteth-20, Laureth-4, Laureth-23.

Le ou les tensioactifs non-ioniques sont présents de préférence à des concentrations allant de 5 à 20% en poids et plus préférentiellement de 7 à 12% en poids par rapport au poids total de la composition.

L'agent auto-moussant est choisi de préférence parmi les hydrocarbures volatils et les hydrocarbures volatils halogénés ayant un point d'ébullition suffisamment bas pour permettre à ces derniers de s'évaporer et de mousser le gel à l'application sur la peau et un point d'ébullition suffisamment haut pour éviter de produire une mousse prématurément. Le point d'ébullition de l'agent auto-moussant varie de préférence de -20 à 40°C. L'agent auto-moussant est choisi de préférence de telle sorte à former une pression de vapeur à 20°C de 3 à 20 psig et de préférence de 5 à 15 psig. Les agents auto-moussants utilisables selon l'invention sont choisis parmi les hydrocarbures aliphatiques en C₄-C₆ tels que n-pentane, isopentane, néopentane, n-butane, isobutane et leurs mélanges. Plus préférentiellement, on utilisera un mélange isopentane/isobutane dans un rapport en poids allant de 1/1 à 3/1. L'agent auto-moussant est de préférence présent à des concentrations allant de 1 à 8% en poids et plus préférentiellement de 2 à 5% en poids par rapport au poids total de la composition.

Les compositions convenant à l'invention peuvent contenir en plus un agent gélifiant et par exemple contenir au moins un hydrocarbure liquide non volatil. Les termes « volatil » et « liquide » signifient que ces matériaux sont liquides à température ambiante et ont un point d'ébullition au dessus de 200 °C. Parmi ces hydrocarbures liquides, on peut citer les huiles minérales, les liquides aliphatiques ramifiés. Ces liquides ont de préférence de 16 à 48 atomes de carbone, plus préférentiellement de 20 à 40 atomes de carbone et une viscosité cinétique (mesurée selon la norme ASTM D445) de 5 à 100 cst et plus préférentiellement de 10 à 70 cst à 40 °C. Les hydrocarbures liquides non-volatils préférentiels sont choisis parmi les huiles minérales ayant une viscosité cinétique de 10 à 70 cst, les polyisobutènes hydrogénés de poids moléculaire de 320 à 420 et leurs mélanges. Le ou les hydrocarbures liquides non volatils sont de préférence présents à des concentrations inférieures ou égales à 10% et de préférence inférieures ou égales à 7% en poids par rapport au poids total de la composition.

Les compositions convenant à l'invention peuvent également contenir un agent gélifiant auxiliaire hydrosoluble ou un agent épaississant pour améliorer la consistance et la stabilité du gel ou pour ajuster la viscosité.

Parmi ces agents gelifiants auxiliaires, on peut citer les polymères hydroxyalkylcelluloses comme hydroxyethylcellulose ou hydroxypropylcellulose (produits vendus respectivement sous la dénomination commerciale NATROSOL ou KLUCEL) ; les copolymères d'acide acrylique et de polyallylsucrose (produits vendus sous la dénomination commerciale CARBOPOL) ; la carboxymethylcellulose et la cellulose methyl ether (produits vendus sous la dénomination commerciale METHOCEL) ; les gommes naturelles ou synthétiques, les amidons. Les agents gelifiants auxiliaires ou épaississants sont de préférence présents à des concentrations allant de 0,01 à 5% en poids, plus préférentiellement de 0,05 à 2% en poids et encore plus préférentiellement de 0,01 à 2% en poids par rapport au poids total de la composition.

Les compositions convenant à l'invention peuvent contenir en plus un polyol à chaîne courte pour améliorer les qualités de mousse et/ou la stabilité de la composition. Le ou les polyols sont de préférence présents à des concentrations inférieures à 10% en poids et plus préférentiellement allant de 0,25 à 5% en poids par rapport au poids total de la composition. Parmi les polyols utilisables ont peut citer la glycérine, le propylène glycol ou leurs mélanges.

Les compositions convenant à l'invention peuvent contenir en plus une variété d'ingrédients cosmétiques classiques pour améliorer les qualités esthétiques et les performances de ces compositions.

Les compositions convenant à l'invention peuvent également contenir en plus un polymère cationique conditionneur pour améliorer la lubricité et le toucher de la peau après rasage. On peut citer par exemple les sels d'ammonium quaternaires de l'hydroxyyethylcellulose comme Polyquaternium-10, Polyquaternium-24.

On peut également citer les polymères cationiques suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM ;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP ;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF;
Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Le ou les polymères cationiques conditionneurs sont présents de préférence à des concentrations allant de 0,05 à 2% en poids, plus préférentiellement allant de 0,1 à 1% en poids par rapport au poids total de la composition.

D'autres additifs peuvent également être utilisés dans les compositions convenant à l'invention comme
- les humectants comme le sorbitol ;
- les émollients tels que les esters gras comme l'isopropyl myristate, le decyl oleate, le 2-ethyhexyl palmitate, le PEG-7 Glyceryl Cocoate et le Glyceryl Linoleate ; les éthers gras propoxylés comme PPG-10 Cetyl Ether et PPG-11 Stearyl Ether ; les di- ou triglycérides comme la lécithine, le mélange de triglycérides caprique/caprylique, le PEG-10 Soy Sterol, les huiles végétales.
- les agents rafraichissants et les agents apaisants comme le menthol, l'aloe, l'allantoine, la lanoline, le bisabolol, l'acide hyaluronique ;
- les lubrifiants comme les polyethyleneglycols (ie PEG-14M, PEG-23M), les tensioactifs fluorés, les silicones (ie : dimethicone, dimethiconol, dimethicone copolyol, stearyl dimethicone, cetyl dimethicone copolyol, cyclomethicone, etc...)
- les vitamines incluant les précurseurs et les dérivés comme le panthenol, le tocopheryl acetate, le niacinamide, le retinyl palmitate, le vitamine A palmitate ;
- les colorants ;
- les parfums ;
- les antioxydants ;
- les antibactériens et/ou les antifongiques ;
- les conservateurs (ie : methylchloroisothiazolinone, methylisothiazolinone, DMDM hydantoine, iodopropinyl butylcarbamate).

Bien entendu, l'homme de l'art veillera à choisir le ou les éventuels composés complémentaires cités ci-dessus et/ou leurs quantités de manière telle que les propriétés avantageuses attachées intrinsèquement aux compositions convenant à l'invention ne soient pas, ou substantiellement pas, altérées par la ou les adjonctions envisagées.

Les compositions convenant à l'invention peuvent être conditionnées dans tout dispositif permettant de délivrer un gel auto-moussant. Par exemple, le dispositif peut être un récipient aérosol avec une séparation telle qu'un piston ou une poche souple pour séparer l'agent auto-moussant de l'agent propulseur nécessaire pour l'expulsion du produit. Le dispositif peut être également un tube souple ; un flacon pompe ou un flacon à paroi déformable.

Les exemples qui suivent servent à illustrer l'invention. Les quantités indiquées sont en % en poids par rapport au poids total de la composition et les noms des composés en noms chimiques ou noms INCI selon les cas.

### EXEMPLES

On prépare les gels sans savon auto-moussants suivants A, et B et C (hors invention) qui sont dans des valves à poche.

| **Ingrédients** | **Composition A** | **Composition B (hors invention)** | **Composition C (hors invention)** |
|---|---|---|---|
| EDTA | 0,05 | 0,05 | 0,05 |
| Triéthanolamine | 1,3 | 1,3 | 1,3 |
| Paraffinum Liquidum | 1,25 | 1,25 | 1,25 |
| Alcool myristique | 2,00 | 2,00 | 2,00 |
| Hydroxyethyllcellulose | 0,20 | 0,20 | 0,20 |
| Hydroxypropyl guar (JAGUAR HP 105 de Rhodia) | 0,15 | 0,15 | 0,15 |
| Gomme de xanthane | 0,15 | 0,15 | 0,15 |
| Glycérine | 4,00 | 4,00 | 4,00 |
| Acide myristique | 1,00 | 1,00 | 1,00 |
| Oleth-30 | 8,00 | 8,00 | 8,00 |
| PEG-14M (Polyethylene Glycol 14.000 OE) | 0,20 | 0,20 | 0,20 |
| Sodium Laureth Sulfate à 2,2 OE en solution aqueuse à 70% | - | - | 5,36 (3,75 en matière active) |
| Lauryl Bétaine (and) Sodium Chloride en solution aqueuse à 30% | 12,50 (3,75 en matière active) | - | - |
| Stearoyl sarcosine (and) Myristoyl sarcosine (75%/25%) | 3,00 | 3,00 | 3,00 |
| Isopentane et Isobutane (75/25) | 2,75 | 2,75 | 2,75 |
| Eau | qsp 100 | qsp 100 | qsp 100 |
| **Rigidité du Gel (en grammes)** | **63 g** | **4,7 g** | **35 g** |

On mesure à 25°C la rigidité de chaque gel au moyen d'un analyseur de texture TA XT2i fabriqué par la société THERMO muni d'un cylindre SMS P/0-5 HS 0.5 inch diameter Hemi Spherical Delrin Cylinder Probe. On mesure la rigidité (exprimée en grammes) de chaque produit en compression par ledit cylindre à une vitesse de 2 mm/s sur une distance de 25 mm. On observe que la composition B de l'art antérieur ne contenant ni de tensioactif amphotère ni de tensioactif anionique ne permet pas d'obtenir un gel rigide contrairement à la composition A convenant à l'invention contenant un tensioactif amphotère. On observe que la composition C de l'art antérieur contenant à la place du tensioactif amphotère un tensio-actif anionique à la même concentration en matière active conduit à une mousse sensiblement moins rigide que la composition A convenant à l'invention.

## Revendications

1. Dispositif de délivrance d'un gel auto-moussant sans savon comprenant :
(i) un récipient aérosol avec une séparation telle qu'un piston ou une poche souple pour séparer l'agent auto-moussant de l'agent propulseur nécessaire pour l'expulsion du produit
(ii) un tube souple ;
(iii) un flacon pompe ou
(iv) un flacon à paroi déformable, et
un gel auto-moussant sans savon comprenant dans un milieu cosmétiquement acceptable :
a) au moins une phase aqueuse
b) au moins une N-acylsarcosine où le radical acyle est en C10-C20 dans une quantité inférieure à 4% en poids par rapport au poids total de la composition ;
c) au moins une base minérale ou organique dans une quantité suffisante pour solubiliser la N-acylsarcosine et produire un pH de 4 à 8 ;
d) au moins un tensioactif amphotère ou zwittérionique ;
e) au moins un tensioactif non-ionique
f) au moins un agent automoussant choisi parmi les hydrocarbures volatils et les hydrocarbures volatils halogénés de point d'ébullition allant de -20 à 40 °C.

2. Dispositif selon la revendication 1, dans lequel la N-acylsarcosine est choisie parmi stéaroyl sarcosine, myristoyl sarcosine et leurs mélanges.

3. Dispositif selon l'une des revendications 1 et 2, dans lequel la ou les N-acylsarcosines sont présentes à des teneurs allant de 2,5 à 3,5% par rapport au poids total de la composition.

4. Dispositif selon l'une quelconque des revendications 1 à 3, dans lequel la base est la triéthanolamine.

5. Dispositif selon l'une quelconque des revendications 1 à 4, dans lequel la sarcosine est neutralisée de 50 à 90%, plus préférentiellement de 60 à 80%.

6. Dispositif selon l'une quelconque des revendications 1 à 5, dans lequel la base est présente à un taux variant de 1 à 6% par rapport au poids total de la composition.

7. Dispositif selon l'une quelconque des revendications 1 à 6, dans lequel la phase aqueuse des compositions selon l'invention représente de préférence de 65 à 85% en poids du poids total de la composition et plus préférentiellement de 70 à 80% en poids.

8. Dispositif selon l'une quelconque des revendications 1 à 7, dans lequel le ou les tensioactifs non-ioniques sont choisis parmi :
- les alcools gras dont la chaîne grasse est en C₈-C₂₀, plus préférentiellement dont la chaîne grasse est en C₁₂-C₁₈ ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène ;
et leurs mélanges

9. Dispositif selon l'une quelconque des revendications 1 à 8, dans lequel le ou les tensioactifs amphotères et zwitterioniques conformes à l'invention peuvent être choisi parmi les alkylbétaïnes, les N-alkylamidobétaïnes et leurs dérivés, les sultaïnes, les alkyl-polyaminocarboxylate (APAC), les alkylamphoacétates et leurs mélanges.

10. Dispositif selon la revendication 9, dans lequel le ou les tensioactifs amphotères et zwitterioniques sont choisis parmi les alkylbétaines.

11. Dispositif selon la revendication 10, dans lequel l'alkylbétaine est la lauryl bétaine et plus préferentiellement la lauryl bétaine sous forme de solution aqueuse à 30% en mélange avec du chlorure de sodium.

12. Dispositif selon l'une des revendications 1 à 11, dans lequel l'agent auto-moussant est choisi parmi les hydrocarbures aliphatiques en C₄-C₆ tels le n-pentane, isopentane, néopentane, n-butane, isobutane et leurs mélanges.

13. Procédé de rasage consistant à appliquer sur la surface de la peau à raser un gel auto-moussant tel que défini dans l'une quelconque des revendications précédentes délivré au moyen d'un dispositif de délivrance tel que revendiqué dans l'une quelconque des revendications 1 à 12 puis à raser les poils au moyen d'un rasoir.

14. Procédé de nettoyage de la peau et plus particulièrement du visage consistant à appliquer sur la surface un gel auto-moussant tel que défini dans l'une quelconque des revendications précédentes délivré au moyen d'un dispositif de délivrance tel que revendiqué dans l'une quelconque des revendications 1 à 12 puis à effectuer un rinçage à l'eau.

## Patentansprüche

1. Abgabevorrichtung für ein schaumbildendes Gel ohne Seife, umfassend:
(i) einen Aerosolbehälter mit einer Trennung wie einem Kolben oder einem flexiblen Beutel zum Trennen des schaumbildenden Mittels von dem zum Ausstoßen des Produkts notwendigen Treibmittel,
(ii) eine Quetschtube;
(iii) eine Pumpflasche oder
(iv) eine Flasche mit verformbarer Wand, und
ein schaumbildendes Gel ohne Seife, das in einem kosmetisch akzeptablen Medium umfasst:
a) mindestens eine wässrige Phase,
b) mindestens ein N-Acylsarcosin, bei dem der Acylrest mit C₁₀ bis C₂₀ in einer Menge unter 4 Gewichts-% bezogen auf das Gesamtgewicht der Zusammensetzung vorliegt;
c) mindestens eine mineralische oder organische Base in einer ausreichenden Menge zum Solubilisieren von N-Acylsarcosin und Erzeugen eines pH von 4 bis 8;
d) mindestens ein amphoteres oder zwitterionisches Tensid;
e) mindestens ein nicht-ionisches Tensid,
f) mindestens ein schaumbildendes Mittel, ausgewählt aus flüchtigen Kohlenwasserstoffen und halogenierten flüchtigen Kohlenwasserstoffen mit einem Siedepunkt zwischen -20 und 40 °C.

2. Vorrichtung nach Anspruch 1, wobei das N-Acylsarcosin aus Stearoylsarcosin, Myristoylsarcosin und ihren Mischungen ausgewählt ist.

3. Vorrichtung nach Anspruch 1 oder 2, wobei das oder die N-Acylsarcosine mit einem Gehalt von 2,5 bis 3,5 % bezogen auf das Gesamtgewicht der Zusammensetzung vorliegt bzw. vorliegen.

4. Vorrichtung nach einem der Ansprüche 1 bis 3, wobei die Base Triethanolamin ist.

5. Vorrichtung nach einem der Ansprüche 1 bis 4, wobei das Sarcosin zu 50 bis 90%, bevorzugter zu 60 bis 80% neutralisiert ist.

6. Vorrichtung nach einem der Ansprüche 1 bis 5, wobei die Base in einem veränderlichen Anteil von 1 bis 6% bezogen auf das Gesamtgewicht der Zusammensetzung vorliegt.

7. Vorrichtung nach einem der Ansprüche 1 bis 6, wobei die wässrige Phase der erfindungsgemäßen Zusammensetzungen vorzugsweise 65 bis 85 Gewichts-% des Gesamtgewichts der Zusammensetzung und bevorzugter 70 bis 80 Gewichts-% ausmacht.

8. Vorrichtung nach einem der Ansprüche 1 bis 7, wobei das oder die nichtionischen Tenside ausgewählt sind aus:
- Fettalkoholen, deren Fettkette C₈ bis C₂₀ umfasst, deren Fettkette bevorzugter C₁₂ bis C₁₈ umfasst;
- Polyoxyethylenethern von Fettalkoholen, deren Fettkette C₈ bis C₂₀ umfasst, deren Fettkette bevorzugter C₁₂ bis C₁₈ umfasst, und die 2 bis 60, bevorzugter 2 bis 30 Ethylenoxydeinheiten aufweisen, sowie ihren Mischungen.

9. Vorrichtung nach einem der Ansprüche 1 bis 8, wobei das oder die amphoteren und zwitterionischen erfindungsgemäßen Tenside ausgewählt sein können aus Alkylbetainen, N-Alkylamidobetainen und ihren Abkömmlingen, Sultainen, Alkylpolyaminocarboxylaten (APAC), Alkylamphoacetaten und ihren Mischungen.

10. Vorrichtung nach Anspruch 9, wobei das oder die amphoteren und zwitterionischen Tenside aus Alkylbetainen ausgewählt sind.

11. Vorrichtung nach Anspruch 10, wobei das Alkylbetain Laurylbetain und bevorzugter Laurylbetain in Form einer wässrigen 30%-igen Lösung gemischt mit Natriumchlorid ist.

12. Vorrichtung nach einem der Ansprüche 1 bis 11, wobei das schaumbildende Mittel ausgewählt ist aus aliphatischen Kohlenwasserstoffen mit C₄ bis C₆, beispielsweise n-Pentan, Isopentan, Neopentan, n-Butan, Isobutan und ihren Mischungen.

13. Rasurverfahren, das darin besteht, auf die zu rasierende Hautoberfläche ein schaumbildendes Gel nach einem der vorhergehenden Ansprüche aufzutragen, das mit einer in einem der Ansprüche 1 bis 12 beanspruchten Abgabevorrichtung abgegeben wird, und anschließend die Haare mit einem Rasierer abzurasieren.

14. Verfahren zum Reinigen der Haut und insbesondere des Gesichts, das darin besteht, auf die Oberfläche ein schaumbildendes Gel nach einem der vorhergehenden Ansprüche aufzutragen, das mit einer in einem der Ansprüche 1 bis 12 beanspruchten Abgabevorrichtung abgegeben wird, und anschließend mit Wasser zu spülen.

## Claims

1. Device for delivering a soap-free, self-foaming gel comprising:
(i) an aerosol container with a separation such as a piston or a flexible pocket to separate the self-foaming agent from the propellant that is necessary for ejecting the product;
(ii) a flexible tube;
(iii) a pump dispenser or
(iv) a dispenser having deformable walls, and
a soap-free, self-foaming gel comprising, in a cosmetically acceptable medium:
a) at least one aqueous phase;
b) at least one N-acylsarcosine where the acyl radical is a C₁₀-C₂₀ radical in an amount less than 4 wt% relative to the total weight of the composition;
c) at least one mineral or organic base in an amount sufficient to dissolve the N-acylsarcosine and produce a pH of 4 to 8;
d) at least one amphoteric or zwitterionic surfactant;
e) at least one non-ionic surfactant; and
f) at least one self-foaming agent chosen from volatile hydrocarbons and halogenated volatile hydrocarbons, having a boiling point ranging from -20 to 40°C.

2. Device according to Claim 1, in which the N-acylsarcosine is chosen from stearoyl sarcosine, myristoyl sarcosine and mixtures thereof.

3. Device according to either of Claims 1 and 2, in which the N-acylsarcosine or N-acylsarcosines are present at content levels ranging from 2.5 to 3.5% relative to the total weight of the composition.

4. Device according to any one of Claims 1 to 3, in which the base is triethanolamine.

5. Device according to any one of Claims 1 to 4, in which the sarcosine is 50 to 90%, more preferentially 60 to 80%, neutralized.

6. Device according to any one of Claims 1 to 5, in which the base is present at a level varying from 1 to 6% relative to the total weight of the composition.

7. Device according to any one of Claims 1 to 6, in which the aqueous phase of the compositions according to the invention preferably represents from 65 to 85 wt%, and more preferentially from 70 to 80 wt%, of the total weight of the composition.

8. Device according to any one of Claims 1 to 7, in which the non-ionic surfactant or surfactants are chosen from:
- fatty alcohols having a C₈-C₂₀ fatty chain, more preferentially having a C₁₂-C₁₈ fatty chain;
- polyoxyethylenated ethers of fatty alcohols having a C₈-C₂₀ fatty chain, more preferentially having a C₁₂-C₁₈ fatty chain and having 2 to 60, more preferentially 2 to 30 ethylene oxide units,
and mixtures thereof.

9. Device according to any one of Claims 1 to 8, in which the amphoteric and zwitterionic surfactant or surfactants according to the invention may be chosen from alkylbetaines, N-alkylamidobetaines and derivatives thereof, sultaines, alkylpolyaminocarboxylate (APAC), alkylamphoacetates and mixtures thereof.

10. Device according to Claim 9, in which the amphoteric and zwitterionic surfactant or surfactants are chosen from alkylbetaines.

11. Device according to Claim 10, in which the alkylbetaine is laurylbetaine and more preferably laurylbetaine in the form of a 30% aqueous solution as a mixture with sodium chloride.

12. Device according to any one of Claims 1 to 11, in which the self-foaming agent is chosen from aliphatic C₄-C₆ hydrocarbons such as n-pentane, isopentane, neopentane, n-butane, isobutane and mixtures thereof.

13. Shaving method consisting in applying a self-foaming gel as defined in any one of the preceding claims delivered by means of a delivery device as claimed in any one of Claims 1 to 12 to the surface of the skin to be shaved, then in shaving the hairs using a razor.

14. Method for cleansing the skin and more particularly the face, consisting in applying a self-foaming gel as defined in any one of the preceding claims delivered by means of a delivery device as claimed in any one of Claims 1 to 12 to the surface, then in rinsing with water.
